(19) Europäisches Patentamt
European Patent Office
Office européen des brevets

(11) Veröffentlichungsnummer: **0 345 719 B1**

(12) **EUROPÄISCHE PATENTSCHRIFT**

(45) Veröffentlichungstag der Patentschrift: **29.09.93**

(51) Int. Cl.$^5$: **C07C 69/60**, C07C 69/40, C07C 67/26, C07C 67/08

(21) Anmeldenummer: **89110194.1**

(22) Anmeldetag: **06.06.89**

Verbunden mit 89906769.8/0420884
(europäische
Anmeldenummer/Veröffentlichungsnummer)
durch Entscheidung vom 11.12.92.

# (54) Verbindungen mit mindestens drei funktionellen Estergruppen und Verfahren zu ihrer Herstellung.

(30) Priorität: **10.06.88 DE 3819738**

(43) Veröffentlichungstag der Anmeldung:
**13.12.89 Patentblatt 89/50**

(45) Bekanntmachung des Hinweises auf die Patenterteilung:
**29.09.93 Patentblatt 93/39**

(84) Benannte Vertragsstaaten:
**AT BE CH DE ES FR GB GR IT LI LU NL SE**

(56) Entgegenhaltungen:
**GB-A- 1 060 750**
**US-A- 3 960 935**
**US-A- 4 316 987**

(73) Patentinhaber: **Chemische Fabrik Stockhausen GmbH**
**Bäkerpfad 25**
**D-47805 Krefeld(DE)**

(72) Erfinder: **Peppmöller, Reinmar, Dr.-Dipl.-Chem.**
**Kemmerhofstrasse 189**
**D-4150 Krefeld(DE)**

(74) Vertreter: **Klöpsch, Gerald, Dr.-Ing. Patentanwalt**
**An Gross St. Martin 6**
**D-50667 Köln (DE)**

EP 0 345 719 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Estergruppen enthaltende Verbindungen mit mindestens drei Esterfunktionen, Verfahren zu ihrer Herstellung und die Verwendung der Produkte.

Monoester von Dicarbonsäuren sind bekannt. Bei dem Versuch, diese Verbindungen mit Di- oder Polyolen zu kondensieren, kommt es zu der Bildung von Polykondensaten und es gelingt nicht, niedermolekulare Verbindungen in einer technisch verwertbaren Reaktion zu erhalten.

In der GB-PS 1,379,335 ist der Versuch der Herstellung von Emulsionsstabilisatoren durch Umsetzung von ungesättigten Glycidylestern mit Dicarbonsäuremonoestern beschrieben. Die langwierige Umsetzung führt infolge der Tendenz zur Umlagerung der entstandenen Produkte und ihrer Temperaturempfindlichkeit zu polymerhaltigen bzw. instabilen Endprodukten, die eine wirtschaftliche technische Nutzung nicht ermöglichen.

Auch bei der Alkoxylierung von Bernsteinsäure mit Ethylenoxid wird ein zur Umlagerung neigendes Bis-(hydroxy-ethyl)-succinat erhalten (DE-P 544 288).

Aus der GB-PS 1,060,175 sind Ethylenglykol-bisalkylfumarate der Formel

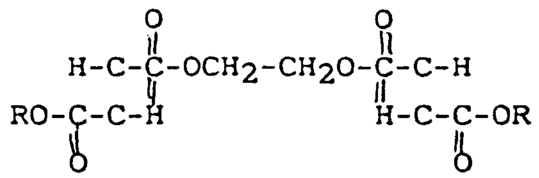

bekannt, die in sehr geringer Ausbeute als unerwünschtes Nebenprodukt bei der Umsetzung von Fumarsäureestern mit Epoxyden zu 2-Hydroxyethylfumaraten entstehen.

Ausgehend von diesem Stand der Technik liegt der vorliegenden Erfindung die Aufgabe zugrunde, ausgehend von Dicarbonsäuremonoestern stabile, mehrere Esterfunktionen enthaltende Verbindungen sowie ein Verfahren zu ihrer Herstellung bereitzustellen.

Diese Aufgabe wird gelöst durch die Synthese von Estergruppen enthaltenden Verbindungen mit mindestens drei funktionellen Estergruppen, im folgenden entsprechend der Bezeichnung für m = 1 als "Triester" bezeichnet, der allgemeinen Formel

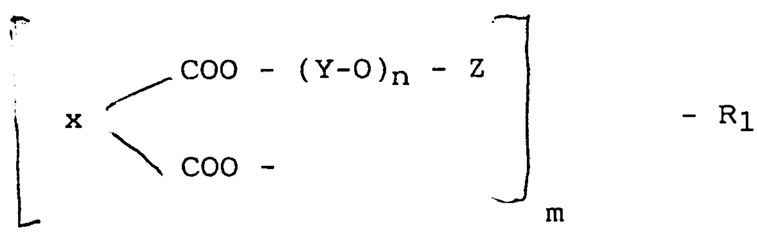

in der die verwendeten Abkürzungen die folgende Bedeutung haben:

x = $-CH_2-CH_2-$ oder $-CH=CH-$,

Y = bivalenter, gesättigter aliphatischer Kohlenwasserstoffrest, der ggfs. mit einer oder mehreren veretherten oder veresterten Hydroxygruppen substituiert ist,

Z = Acylrest -CO-R einer ein- oder zweibasigen, gesättigten oder ungesättigten aliphatischen, cycloaliphatischen oder aromatischen Carbonsäure mit 2 bis 22 C-Atomen, die gegebenenfalls einen gesättigten oder ungesättigten $C_4$-$C_{16}$-Kohlenwasserstoffrest als Substituent trägt,

$R_1$ = Rest eines ein- oder mehrwertigen gesättigten oder ungesättigten aliphatischen, cycloaliphatischen oder aromatischen Alkohols oder eine die Alkoholfunktion in der Estergruppierung übernehmende Gruppe aus einem Alkylenoxid-Addukt,

n = 1 bis 3

m = 1 bis 3, ausgenommen Ethylenglykol bis(alkylfumarate der Formel

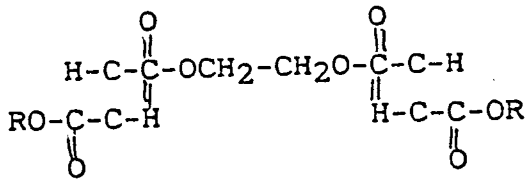

in der R eine Alkylgruppe bedeutet.

Es wurden 2 Synthesewege gefunden.

Der erste Syntheseweg umfaßt die Umsetzung eines Dicarbonsäuremonoesters mit einem Epoxid, d.h. eine Alkoxylierung der Carbonsäuregruppe, wobei das als Zwischenprodukt entstehende Dicarbonsäuremonoester-Alkoxylat (Dicarbonsäure-Hydroxyalkyl-Alkyl-Diester),das thermisch instabil ist, anschließend acyliert und dadurch in die Form des stabilen Triesters überführt wird.

Die als Zwischenprodukt entstehenden Dicarbonsäuremonoester-Alkoxylate haben die allgemeine Formel:

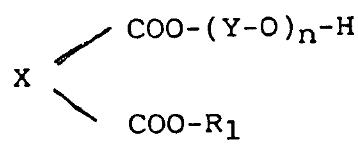

Längeres Verweilen des gebildeten Zwischenproduktes bei erhöhten Temperaturen begünstigt Umlagerungen. Damit ist die Anlagerung mehrerer Mole Alkylenoxid an den Dicarbonsäuremonoester wegen der dafür erforderlichen höheren Reaktionszeit nur in Grenzen möglich. Erfindungsgemäß werden 1 bis 3 Mol angelagert.

Eine Restmenge von nicht umgesetztem Dicarbonsäuremonoester im Reaktionsgemisch nach Alkoxylierung übt einen stabilisierenden Einfluß auf den gebildeten Hydroxyalkyl-Alkyl-Mischester aus. Vorzugsweise beträgt die Säurezahl des Alkoxylierungsgemisches mindestens 5; bei Werten unter 5 treten erhebliche Druckschwankungen und Umlagerungsreaktionen auf.

Bei Anlagerung von 1 bis 3 Mol Alkylenoxid beträgt die Säurezahl vorzugsweise mindestens 10.

Die auf diese Weise hergestellten Hydroxyalkyl-alkyl-Dicarbonsäureester weisen gegenüber dem Dicarbonsäuremonoester einen niedrigeren Schmelzpunkt und eine geringere Wasserlöslichkeit, insbesondere im neutralen bis schwach alkalischen Bereich auf. Dies ermöglicht oftmals eine Reinigung des Hydroxyalkylesters mit Wasser oder einer wässrigen Salzlösung.

Eine längere Lagerung der Hydroxyalkylester ist, auch wenn sie ausgewaschen und katalysatorfrei sind, vorzugsweise bei Temperaturen unter 0 °C möglich. Versuche zur weitergehenden Reinigung des Zwischenprodukts durch thermische Prozesse, wie z.B. fraktionierte Destillation, führen zu unerwünschten polymerprodukten und Abspaltung von Alkohol und Glykolen. Vorzugsweise wird das Zwischenprodukt daher sofort weiterverarbeitet.

Die zur Herstellung der als Ausgangsprodukt dienenden Dicarbonsäuremonoester eingesetzten Dicarbonsäuren können aliphatische, gesättigte oder ungesättigte Säuren sein. Wesentliche Vertreter der Dicarbonsäuren sind Bernstein-, Malein-, Fumarsäure.

Anhydridbildende Säuren sind, da die Herstellung der Monoester am einfachsten aus den Anhydriden der Dicarbonsäure erfolgt, bevorzugt.

Als Alkoholkomponente zur Bildung des Dicarbonsäuremonoesters können beliebige ein- oder mehrwertige gesättigte oder ungesättigte primäre, sekundäre oder tertiäre Alkohole verwendet werden.

Als wichtige Vertreter sind zu nennen: Methanol, Ethanol, Propanol, Butanol, Pentanol, Hexanol, Heptanol, Octanol, Nonanol, Decanol, Undecanol, Dodecanol, Eicosanol, Cetylalkohol, Myricylalkohol, Ethylenglykol, Propylenglykol, Butylenglykol, Hexylenglykol, Glycerin, Trimethylolpropan, Sorbit, Sorbitan, Diethylenglykol, Polyethylenglykol, Dipropylenglykol, Polypropylenglykol, Ethylenglykolmonomethylether, Diethylenglykolmonomethylether, monoendverschlossenes Polyethylenglykol, monoendverschlossenes Polypropylenglykol, Polyrandomaddukte von Ethylen- und Propylenoxid, Fettalkoholoxethylate, Fettalkoholpropyoxylate, Nonylphenoloxethylate, Nonylphenolpropoxylate, Allylalkohol, Zimtalkohol und Benzylalkohol.

Ebenso sind grundsätzlich alle Alkylenoxidaddukte, die aciden Wasserstoff enthalten, einsetzbar.

Der Umsetzungsgrad der Esterbildung kann in üblicher Weise über eine Säure- bzw. OH-Zahl-Bestimmung verfolgt werden.

Die Mengenverhältnisse zwischen Alkohol und Dicarbonsäure bzw. -anhydrid werden entsprechend der stöchiometrischen Umsetzung zum Halbester gewählt. Bei mehrwertigen Alkoholen sind auch Teilveresterungen möglich. Die Reaktion wird bei Temperaturen unter 120 °C, vorzugsweise unter 90 °C durchgeführt, kann jedoch auch, falls der Schmelzpunkt des Anhydrids höher liegt, kurzzeitig bis zu dieser Temperatur erhöht werden. Bei Verwendung niedrig siedender Alkohole oder sublimierender Substanzen wird im Druckgefäß gearbeitet. Zweckmäßigerweise werden die hergestellten Monoester wegen ihrer Thermoinstabilität direkt in noch heißem Zustand weiterverarbeitet.

Zur Bildung der Hydroxyalkylester können alle Verbindungen mit Epoxidstruktur eingesetzt werden. Als bevorzugte Vertreter der Stoffgruppe der Epoxide werden genannt: Ethylenoxid, Propylenoxid, 1,2-Epoxibutan, 2,3-Epoxi-1-propanol, Glycidylacrylat, Glycidylmethacrylat, Glycidylallylether, 1,3-Butandienmonooxid, Styroloxid, Epoxibuttersäureethylester, 1,2-Epoxicyclodeca-5,9-dien, 1,2-Epoxicyclododecan, 1,2-Epoxicyclohexan, exo-2,3-Epoxinorbornan, 2,3-Epoxipropyl-4-methoxiphenylether.

Eine unter sterischen und/oder kinetischen Gesichtspunkten ungünstige Wahl der Reaktionspartner kann zu einer Beeinträchtigung der Reaktionszeiten und/oder Ausbeuten führen.

Durch Umsetzung des thermisch instabilen Zwischenproduktes mit einem Acylierungsmittel werden erfindungsgemäß die thermisch stabilen Triester erhalten. Vorzugsweise werden zur Acylierung Carbonsäureanhydride, Carbonsäurechloride oder Ketene eingesetzt. Die Acylierung wird im Anschluß an die Epoxidanlagerung an den Dicarbonsäuremonoester bei der gleichen Temperatur durchgeführt.

Umesterungen bzw. Polymerbildungen werden durch die Acylierung der OH-Gruppen verhindert.

Art und Länge des Kohlenwasserstoffrestes im Acylierungsmittel üben einen stärkeren Einfluß auf die physikalischen Eigenschaften des Mischesters aus als die des Alkoholrestes des Dicarbonsäuremonoesters. So stellt bspw. das Acetyl-oxiethyl-benzylmaleinat bei Raumtemperatur eine niedrigviskose, wasserklare Flüssigkeit dar, während das Benzoyl-oxyethyl-methylmaleinat unter den Bedingungen der Destillation zum Fumarsäureester umgelagert wird und unterhalb von 51 °C ein weißes Kristallisat bildet.

Bei einer Ausführungsform des erfindungsgemäßen Verfahrens wird eine Umsetzung der Hydroxialkylalkyl-ester mit Dicarbonsäureanhydriden ähnlich der oben beschriebenen Bildung der Monoester durchgeführt.

Bei dieser Synthesevariante weist das erhaltene Triester-Molekül,sowohl die Struktur eines Dicarbonsäure-Diesters als auch die eines Dicarbonsäure-Monoesters auf. Es können hierbei sowohl gleiche als auch verschiedene Dicarbonsäure-Grundkörper in einem Molekül vereinigt werden. Die Carbonsäureanhydride werden nach den gleichen Kriterien ausgewählt wie bei der Dicarbonsäuremonoester-Herstellung beschrieben.

Eine direkte Möglichkeit zur Synthese der Triester ist die Umsetzung von Dicarbonsäuremonoester mit Alkylencarbonaten, wie z.B. Ethylencarbonat (1,3-Dioxolan-2-on) bzw. Propylencarbonat(4-Methyl-1,3-dioxolan-2-on) bei gleichzeitiger Anwesenheit eines Acylierungsmittels.

Bei diesem Syntheseweg wird die Stufe des thermisch instabilen Zwischenproduktes, der Hydroxialkylalkyl-dicarbonsäurediester übersprungen. Die damit möglichen erhöhten Reaktionstemperaturen von oberhalb 120 °C ermöglichen einerseits höhere Reaktionsgeschwindigkeiten und andererseits bei Anwendung von niedrigsiedenden Anhydriden als Acylierungsmittel, wie z.B. Essigsäureanhydrid, die direkte Abdestillation der freigesetzten Monocarbonsäure. Dieses Verfahren ist insbesondere zur Herstellung kleiner Triestermengen sowie zur Umsetzung von Dicarbonsäuremonoestern mit hohem Schmelzpunkt geeignet, bei denen eine Hydroxialkylierung mit einem Epoxid zu Umlagerungen und Polyesterbildung führen würde.

Besonders bevorzugt ist die Verwendung der erfindungsgemäßen Verfahren auf Alkoxilate.

Die erfindungsgemäßen thermisch stabilen und lagerstabilen Triester weisen vorteilhafte Eigenschaften bzgl. Konsistenz, Geruch, Siedepunkt, Flammpunkt verbunden mit einer einstellbaren Öl- bzw. Wasserverträglichkeit auf.

Durch das Vorhandensein der mindestens drei funktionellen Estergruppen sowie der Möglichkeit, mindestens drei Strukturelemente der erfindungsgemäßen Triester zu variieren, ergibt sich eine Vielzahl möglicher Verbindungen gemäß der Lehre der Erfindung, die als Lösungsmittel bzw. Lösungsvermittler, Weichmacher, Antistatika und Gleitmittel von Interesse sind. Die sulfitierten Triester ungesättigter Carbonsäure besitzen Netzmitteleigenschaften. Die Produkte sind im allgemeinen gut biologisch abbaubar und weisen somit eine hohe Umweltverträglichkeit auf.

Durch Einbau ungesättigter Carbonsäuren und anschließende Sulfitierung wird eine breite Palette umweltschonender Tenside zugänglich gemacht, deren Anwendungsmöglichkeiten vom Hydrophobierungsmittel bis zum Netz- bzw. Waschmittel reicht.

Durch Einbau ungesättigter Gruppen, z.B. Veresterung mit einem ungesättigten Alkohol, wie z.B. Allylalkohol mit einer Dicarbonsäure oder durch Acylierung mit einer ungesättigten Carbonsäure, wie z.B. Acrylsäure, entstehen Polymerbausteine, die die aufgeführten Vorteile bspw. als Permanentweichmacher in einpolymerisierter Form in einem Polymerkörper zur Geltung bringen können.

Die Thermostabilität der erfindungsgemäßen Triester ist so hoch, daß eine Vakuumdestillation auch bei hohen Temperaturen ohne Zersetzung möglich ist, so daß der gewonnene Triester sich über eine Fraktionierkolonne reinigen läßt. Bei der Destillation von ungesättigten Triestern, z.B. den Maleinaten, muß jedoch bei höheren Temperaturen etwa ab 150 °C mit der Entstehung von Isomeren, z.B. den Fumaraten, gerechnet werden, die sich kaum noch destillativ abtrennen lassen. Eine vollständige Umwandlung tritt bei der Destillation des Stearoyl-, Oxiethyl-, Methyl-Maleinats ein (vgl. Beispiel 9).

Die Identifizierung und Charakterisierung der flüssigen, unter Hochvakuum destillierten Triester lassen sich u.a. durch Bestimmung von Dichte d, Brechungsindex n und Molmasse M vornehmen.

Nach der Lorentz-Lorenz-Gleichung

$$R_M = \frac{n^2-1}{n^2+2} \times \frac{M}{d}$$

besteht eine Beziehung zur Molrefraktion ($R_M$), welche eine charakteristische Kennziffer darstellt und sich aus Atom- bzw. Bindungsrefraktionen ("Inkremente") berechnen läßt (J. chem. Soc. 1948, S. 1804-1855 bzw. Römpps Chemie-Lexikon, 8.Aufl., Bd. 5, S. 3534). Für die Primärprodukte, d.h. reinen Triester mit der Epoxid-anlagerungszahl n = 1, ist diese Forderung erfüllt.

Allgemeine Herstellungsvorschrift für die Beispiele 1 bis 8 und 16:

1 Mol Dicarbonsäuremonoester, hergestellt aus äquimolaren Mengen von Dicarbonsäureanhydrid und Alkohol bei 70 °C, wird mit 1,5 Mol eines Epoxids in Gegenwart von 0,5 bis 1,0 Gew.-% Dimethylbenzylamin als Katalysator bei 80 °C im Autoklaven unter Stickstoff erhitzt. Nach einer Reaktionszeit von 1 Std. ist die Säurezahl auf einen Wert unter 15 abgefallen. Zu dem Reaktionsprodukt werden 1 bis 1,5 Mole Carbonsäureanhydrid gegeben und sodann nochmals 1 Std. bei 80 °C nachgerührt. Anschließend wird der Inhalt des Reaktionsgefäßes über eine Fraktionierkolonne unter Hochvakuum destilliert.

Die Tabelle 1 gibt einen Überblick über die eingesetzten Rohstoffe und die Siedepunkte der erhaltenen Triester sowie die Dichten und Brechungsindices bei 20 °C. Oberhalb der aufgeführten Siedetemperaturen (ca. 20 bis 30 °C höher) werden weitere Fraktionen erhalten, die aus einer Mehrfachanlagerung des Epoxids an den Dicarbonsäuremonoester resultieren.

Es wurden folgende erfindungsgemäßen Ester hergestellt:

1) Acetyl-oxiethyl-methylmaleinat
2) Acetyl-oxipropyl-methylmaleinat
3) Acetyl-oxiethyl-ethylmaleinat
4) Acetyl-oxiethyl-2-ethylhexylmaleinat
5) Acetyl-oxiethyl-benzylmaleinat
6) Acetyl-oxiethyl-methylsuccinat
7) Acetyl-oxiethyl-ethylsuccinat
8) Acetyl-oxiethyl-n-propylsuccinat

16) Acetyl-oxiethyl-allylmaleinat

Tabelle 1

| Beispiel | Dicarbonsäurekomponente | Alkohol | Epoxid | Acylierungskomponente | Siedepunkt | Dichte g/ml | Brechungsindex n(20°C) |
|---|---|---|---|---|---|---|---|
| 1. | MSA | Methanol | EO | AcA | 102°C/0,1bar | 1,19 | 1,455 |
| 2. | MSA | Methanol | PO | AcA | 105°C/0,1bar | 1,16 | 1,453 |
| 3. | MSA | Ethanol | EO | AcA | 103°C/0,1bar | 1,16 | 1,454 |
| 4. | MSA | 2-Ethylhexanol | EO | AcA | 126-128°C (0,1 bar) | 1,04 | 1,455 |
| 5. | MSA | Benzylalkohol | EO | AcA | 148°C/0,1bar | 1,17 | 1,439 |
| 6. | BSA | Methanol | EO | AcA | 102°C/0,1bar | 1,17 | 1,439 |
| 7. | BSA | Ethanol | EO | AcA | 103°C/0,1bar | 1,13 | 1,441 |
| 8. | BSA | n-Propanol | EC | AcA | 103°C/0,1bar | 1,11 | 1,440 |
| 9. | MSA | Methanol | EO | Stear. | 220°C/0,3bar | Schmelzpunkt: | 61°C |
| 10. | MSA | Methanol | EO | Benz. | 147°C/0,25bar | Schmelzpunkt: | 51°C |
| 12. | MSA | Diglykol | EO | AcA | Verseifungszahl: 547 | | |
| 13. | MSA | PEG 600 | EO | AcA | Verseifungszahl: 331 | | |
| 14. | MSA | n-Octadecanol | EO | n-Octenylbernsteinsäureanhydrid | | SZ: | 85 |
| 15. | MSA | " | EO | Isododecenylbernsteinsäureanhydrid | | SZ: | 78 |
| 16. | MSA | Allylalkohol | EO | AcA | 106°C/0,15bar | 1,16 | 1,465 |

Abkürzungen: MSA= Maleinsäureanhydrid, BSA= Bernsteinsäureanhydrid, EO= Ethylenoxid, PO= Propylenoxid, EC= Ethylencarbonat, AcA= Essigsäureanhydrid, Stear. = Stearinsäurechlorid, Benz.= Benzylchlorid, PEG 600= Polyethylenglykol der Molmasse 600.

Die Erfindung wird anhand der nachfolgenden Beispiele verdeutlicht:

Beispiel 1:

Acetyl-oxiethyl-methylmaleinat: 780 g Maleinsäuremonomethylester, hergestellt aus 588 g Maleinsäureanhydrid und 192 g Methanol bei 70 °C, wurden mit 396 g Ethylenoxid in Gegenwart von 10 g Dimethylbenzylamin auf 80 °C im Autoklaven unter Stickstoff erhitzt. Nach einer Reaktionszeit von 1 Std. war die Säurezahl auf 16 (0,28 mmol/g) abgefallen. Zu dem Reaktionsprodukt wurden 700 g Essigsäureanhydrid gegeben und sodann nochmals 1 Std. bei 80 °C nachgerührt. Anschließend wurde der Inhalt des Reaktionsgefäßes über eine Fraktionierkolonne unter Hochvakuum destilliert.

Das Produkt ging bei 102 °C (0,1 mbar) über und stellte bei 20 °C eine geruchlose, niedrigviskose Flüssigkeit mit einer Dichte von 1,19 g/ml und einem Brechungsindex von 1,455 dar.

Eine weitere Fraktion wurde bei 125 bis 130 °C (0,1 mbar) erhalten. Hierbei handelte es sich um den acetylierten Diethylenglykolester mit Anteilen vom Triethylenglykolester des Monomethylmaleinates.

Beispiel 2:

Acetyl-oxipropyl-methylmaleinat: Analog zu Beispiel 1 wurden 780 g Maleinsäuremonomethylester mit 504 g 1,2-Epoxipropan in Gegenwart von 10 g Dimethylbenzylamin und anschließend mit 700 g Essigsäureanhydrid umgesetzt. Danach wurde die Reaktionsmischung einer Fraktionierung unterworfen.

Das Produkt hatte einen Siedepunkt von 105 °C (0,1 mbar) und bei 20 °C eine Dichte von 1,16 g/ml bzw. einen Brechungsindex von 1,453.

Zwischen 125 und 130 °C (0,1 mbar) wurde eine weitere Fraktion erhalten, die aus dem acetylierten Dipropylenglykolester mit Anteilen des Tripropylenglykolesters des Monomethylmaleinates bestand.

Beispiel 3:

Acetyl-oxiethyl-ethylmaleinat: 864 g Maleinsäuremonoethylester, hergestellt aus 588 g Maleinsäureanhydrid und 276 g Ethanol bei 70 °C, wurden mit 396 g Ethylenoxid in Gegenwart von 10 g Dimethylbenzylamin auf 80 °C im Autoklaven unter Stickstoff erhitzt. Nach einer Reaktionszeit von 1 Std. wurden 700 g Essigsäureanhydrid zugesetzt und sodann nochmals 1 Std. bei 80 °C nachgerührt.

Das Produkt hatte bei der anschließenden Fraktionierung einen Siedepunkt von 103 °C (0,1 mbar) und stellte bei 20 °C eine geruchlose, niedrigviskose, wasserklare Flüssigkeit mit einer Dichte von 1,16 g/ml und einem Brechungsindex von 1,454 bei dar.

Eine zweite Fraktion wurde bei 125 bis 130 °C erhalten und bestand aus dem acetylierten Diethylenglykol- und Triethylenglykolester des Monoethylmaleinates.

Beispiel 4:

Acetyl-oxiethyl-2-ethylhexylmaleinat: Es wurden 1824 g Maleinsäure-mono-2-ethylhexylester, hergestellt aus 784 g Maleinsäureanhydrid und 1040 g 2-Ethylhexanol, mit 528 g Ethylenoxid in Gegenwart von 20 g Dimethylbenzylamin und anschließend mit 900 g Essigsäureanhydrid bei 80 °C umgesetzt.

Die Destillation der Reaktionsmischung über eine Fraktionierkolonne ergab für das Produkt einen Siedepunkt von 126 bis 128 °C (0,1 mbar). Bei 20 °C lag die Dichte bei 1,04 g/ml und der Brechungsindex bei 1,455. Der Triester stellte eine geruchlose, niedrigviskose Flüssigkeit dar.

Eine zweite Fraktion wurde zwischen 135 und 138 °C erhalten und bestand aus dem acetylierten Diethylen- und Triethylenglykolester des Monomethylmaleinates.

Beispiel 5:

Acetyl-oxiethyl-benzylmaleinat: 824 g Maleinsäure-monobenzylester, hergestellt aus 392 g Maleinsäureanhydrid und 432 g Benzylalkohol, wurden mit 264 g Ethylenoxid in Gegenwart von 10 g Dimethylbenzylamin und anschließend mit 450 g Essigsäureanhydrid bei 80 °C umgesetzt. Nach der Acylierung des Oxethylates wurde der Triester über eine Fraktionierkolonne unter Vakuum destilliert.

Die Verbindung hatte einen Siedepunkt von 148 °C bei 0,1 mbar. Sie zeigte bei 20 °C eine Dichte von 1,19 g/ml und einen Brechungsindex von 1,511. Sie stellte sich als eine geruchlose, niedrigviskose, wasserklare Flüssigkeit dar.

Das oberhalb 160 °C entnommene Destillat enthielt hauptsächlich den acetylierten Diethylenglykol- und Triethylenglykolester des Monobenzylmaleinats/fumarats.

Beispiel 6:

Acetyl-oxiethyl-methylsuccinat: 792 g Bernsteinsäuremonomethylester, hergestellt aus 600 g Bernsteinsäureanhydrid und 192 g Methanol, wurden mit 396 g Ethylenoxid in Gegenwart von 10 g Dimethylbenzylamin und anschließend mit 700 g Essigsäureanhydrid bei 80°C umgesetzt. Nach der Acylierung wurde die Reaktionsmischung unter Hochvakuum destilliert.

Die Verbindung wurde bei 102°C (0,1 mbar) erhalten und stellte bei 20°C eine geruchlose, niedrigviskose, wasserklare Flüssigkeit mit einer Dichte von 1,17 g/ml und einem Brechungsindex von 1,439 dar.

Eine zweite Fraktion wurde bei 125 bis 130°C erhalten, die aus dem acetylierten Diethylenglykol- und Triethylenglykolester des Monomethylsuccinats bestand.

Beispiel 7:

Acetyl-oxiethyl-ethylsuccinat: 876 g Bernsteinsäuremonoethylester, hergestellt aus 600 g Bernsteinsäureanhydrid und 276 g Ethanol, wurden bei 80°C mit 396 g Ethylenoxid unter Stickstoff im Autoklaven in Gegenwart von 10 g Dimethylbenzylamin und anschließend mit 700 g Essigsäureanhydrid bei gleicher Temperatur umgesetzt. Nach der Acetylierung wurde die Reaktionsmischung unter Hochvakuum destilliert.

Die Verbindung hatte einen Siedepunkt von 103°C bei 0,1 mbar und stellte bei 20°C eine geruchlose, niedrigviskose, wasserklare Flüssigkeit mit einer Dichte von 1,13 g/ml und einem Brechungsindex von 1,441 dar.

Eine weitere Fraktion wurde bei 130 bis 135°C erhalten. Sie bestand aus dem acetylierten Diethylen- und Triethylenglykolester des Monoethylsuccinats.

Beispiel 8:

Acetyl-oxiethyl-n-propylsuccinat: 160 g Bernsteinsäuremono--n-propylester, hergestellt aus 100 g Bernsteinsäureanhydrid und 60 g n-Propanol, wurden mit 90 g Ethylencarbonat und 140 g Essigsäureanhydrid bei angeschlossener Fraktionierkolonne zum Sieden erhitzt. Ab 140°C reagierte die Mischung unter Bildung von Essigsäure, die am Kopf der Kolonne abgenommen wurde. Die Temperatur wurde langsam auf 200°C erhöht und noch 1 Std. dort belassen. Danach wurde auf ca. 50°C abgekühlt und die Vakuumdestillation begonnen.

Die Verbindung hatte einen Siedepunkt von 103 bis 104°C bei 0,1 mbar und stellte bei 20°C eine geruchlose, wasserklare, niedrigviskose Flüssigkeit mit einer Dichte von 1,11 g/ml und einem Brechungsindex von 1,440 dar.

Eine weitere Fraktion wurde im Siedebereich von 140 bis 160°C erhalten und bestand aus dem acetylierten Diethylen- und Triethylenglykolester des Mono-n-propylsuccinats.

Beispiel 9:

Stearoyl-oxiethyl-methylfumarat: 390 g Maleinsäuremonomethylester (s. Beispiel 1) wurden bei 80°C mit 198 g Ethylenoxid in Gegenwart von 5 g Dimethylbenzylamin im Autoklaven unter Stickstoff umgesetzt und anschließend mit 910 g Stearinsäurechlorid bei gleicher Temperatur behandelt. Das Reaktionsprodukt wurde unter Hochvakuum destilliert, wobei die Verbindung bei 220°C (0,3 mbar) überging und in der Kühlvorlage oberhalb ihres Schmelzpunktes gehalten wurde. Nach Umkristallisation in Aceton ergab sich für die Verbindung ein Schmelzpunkt von 61°C. Das Aussehen und die Konsistenz der Substanz ähnelten einem Hartwachs.

Beispiel 10:

Benzoyl-oxiethyl-methylfumarat: 780 g Maleinsäuremonomethylester (s. Beispiel 1) wurden mit 396 g Ethylenoxid in Gegenwart von 10 g Dimethylbenzylamin bei 80°C im Autoklaven unter Stickstoff umgesetzt und anschließend 1 Std. lang bei gleicher Temperatur mit 845 g Benzoylchlorid behandelt. Danach wurde der Triester über eine Fraktionierkolonne unter Hochvakuum destilliert und in der Kühlvorlage oberhalb seines Schmelzpunktes gehalten.

Die Verbindung hatte einen Siedepunkt von 147°C bei 0,25 mbar. Nach Umkristallisation in Aceton wurden weiße Nadeln mit einem Schmelzpunkt von 51°C erhalten.

Beispiel 12:

Bis-acetoxiethylmaleinsäure-diethylenglykolester: 1224 g Bis-maleinsäure-diethylenglykol-monoester, hergestellt aus 636 g Diethylenglykol und 588 g Maleinsäureanhydrid bei 70 °C, wurden mit 396 g Ethylenoxid in Gegenwart von 10 g Dimethylbenzylamin im Autoklaven unter Stickstoff bei 80 °C umgesetzt und anschließend mit 700 g Essigsäureanhydrid acyliert. Danach wurde die Reaktionsmischung in ca. 2 l kaltes Wasser gegossen, kräftig durchgeschüttelt und die wäßrige Phase verworfen. Für den wasserunlöslichen Triester wurde eine Verseifungszahl von 547 ermittelt.

Beispiel 13:

Bis-acetoxiethyl-maleinsäure-polyethylenglykol (600)-ester: 1592 g Bismaleinsäure-polyethylenglykol (600)-monoester, hergestellt aus 1200 g Polyethylenglykol (OH-Zahl: 187) und 392 g Maleinsäureanhydrid, wurden mit 198 g Ethylenoxid in Gegenwart von 10 g Dimethylbenzylamin im Autoklaven unter Stickstoff bei 80 °C umgesetzt und anschließend mit 600 g Essigsäureanhydrid acyliert. Danach wurde die Reaktionsmischung in ca. 2 l warmes Wasser (ca. 60 °C) gegossen und kräftig durchgeschüttelt. Nach dem Absitzen wurde die wäßrige Phase verworfen und das restliche Öl getrocknet. Der Polyglykolester hatte eine Verseifungszahl von 331 und einen Trübungspunkt von ca. 53 °C.

Beispiel 14:

n-Octenyl-succinoyl-oxiethyl-octadecylmaleinat: 1083 g n-Octadecylmaleinat, hergestellt aus 789 g Alfol 16/20 (Handelsbezeichnung der Fa. Condea, BRD, für einen Fettalkohol $C_{16-20}$) und 294 g Maleinsäureanhydrid, wurden mit 145 g Ethylenoxid in Gegenwart von 10 g Dimethylbenzylamin bei 80 °C im Autoklaven unter Stickstoff umgesetzt und sodann mit 630 g n-Octenyl-bernsteinsäureanhydrid 1 Std. lang bei gleicher Temperatur nachbehandelt. Anschließend wurde das Reaktionsprodukt mit Wasser (ca. 50 °C) kräftig geschüttelt, abgetrennt und getrocknet. Es resultierte ein hellgelbes Öl mit einer Esterzahl von 4.99 mmol/g und einer Säurezahl von 85. Mit Alkali oder Ammoniak bildete sich spontan ein wasserlösliches Gel.

Beispiel 15:

Isododecenyl-succinoyl-oxiethyl-octadecylmaleinat: Wie in Beispiel 12 beschrieben, wurden 1228 g oxethyliertes Octadecylmaleinat mit 795 g Isododecenyl-bernsteinsäureanhydrid umgesetzt. Nach dem Reinigen mit warmem Wasser und Trocknen wurde ein gelbes Öl mit einer Esterzahl von 4,30 mmol/g und einer Säurezahl von 78 erhalten. Bei Zugabe von Alkali oder Ammoniak war das Produkt unter Gelbildung wasserlöslich.

Beispiel 16:

Acetyl-oxiethyl-allylmaleinat: 936 g Maleinsäuremonoallylester, hergestellt aus 588 g Maleinsäureanhydrid und 348 g Allylalkohol, wurden mit 396 g Ethylenoxid in Gegenwart von 10 g Dimethylbenzylamin und anschließend mit 750 g Essigsäureanhydrid umgesetzt. Danach wurde die Reaktionsmischung einer Fraktionierung unter Vakuum unterworfen. Zur Stabilisierung wurden 500 ppm Phenothiazol verwandt.

Die Verbindung hatte einen Siedepunkt von 106 °C bei 0,15 mbar und wies bei 20 °C eine Dichte von 1,16 g/ml bzw. einen Brechungsindex von 1,465 auf. Sie war wasserklar, dünnflüssig und fast geruchlos.

Eine zweite Fraktion wurde zwischen 120 und 130 °C erhalten. Hierbei handelte es sich um den acylierten Diethylenglykol- und Triethylenglykolester des Monoallylmaleinates.

**Patentansprüche**
**Patentansprüche für folgende Vertragsstaaten : AT, BE, CH, DE, FR, GB, IT, LI, LU, NL, SE**

**1.** Verbindungen mit mindestens drei funktionellen Estergruppen der allgemeinen Formel:

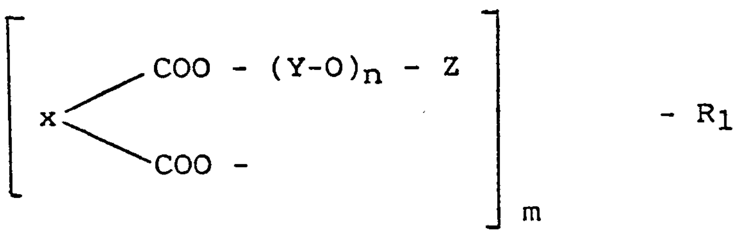

in der die verwendeten Abkürzungen die folgende Bedeutung haben:

x = $-CH_2-CH_2-$ oder $-CH=CH-$,
Y = bivalenter, gesättigter aliphatischer Kohlenwasserstoffrest, der ggfs. mit einer oder mehreren veretherten oder veresterten Hydroxygruppen substituiert ist,
Z = Acylrest -CO-R einer ein- oder zweibasigen, gesättigten oder ungesättigten aliphatischen, cycloaliphatischen oder aromatischen Carbonsäure mit 2 bis 22 C-Atomen, die gegebenenfalls einen gesättigten oder ungesättigten $C_4$-$C_{16}$-Kohlenwasserstoffrest als Substituent trägt,
$R_1$ = Rest eines ein- oder mehrwertigen gesättigten oder ungesättigten aliphatischen, cycloaliphatischen oder aromatischen Alkohols oder eine die Alkoholfunktion in der Estergruppierung übernehmende Gruppe aus einem Alkylenoxid-Addukt,
n = 1 bis 3
m = 1 bis 3, ausgenommen Ethylenglykol bis(alkylfumarate) der Formel

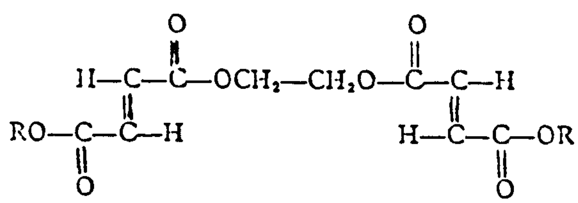

in der R eine Alkylgruppe bedeutet.

**2.** Verbindungen nach Anspruch 1, wobei n einen Wert von 1 bis 3 aufweist.

**3.** Verbindungen nach Anspruch 1 oder 2, wobei Y für eine durch Anlagerung von Alkylen- oder Cycloalkylenoxid gebildete gesättigte Gruppierung steht, die ggfs. einen oder mehrere aliphatische, aromatische oder cycloaliphatische Substituenten oder eine oder mehrere veretherte oder veresterte Hydroxygruppen trägt.

**4.** Verfahren zur Herstellung von Verbindungen nach Anspruch 1, dadurch gekennzeichnet, daß ein Dicarbonsäuremonoester mit einem Epoxid umgesetzt und die Zwischenstufe des Hydroxyalkyl-Alkyl-Dicarbonsäurediesters acyliert wird.

**5.** Verfahren nach Anspruch 4, dadurch gekennzeichnet, daß die Umsetzung des Dicarbonsäuremonoesters mit dem Epoxid bei Temperaturen zwischen 50 und 120 °C durchgeführt wird.

**6.** Verfahren nach Anspruch 5, dadurch gekennzeichnet, daß die Umsetzung des Dicarbonsäuremonoesters mit dem Epoxid bei Temperaturen zwischen 60 und 90 °C durchgeführt wird.

7. Verfahren zur Herstellung von Verbindungen nach Anspruch 1, dadurch gekennzeichnet, daß ein Dicarbonsäuremonoester mit einem Alkylencarbonat in Gegenwart eines Acylierungsmittels umgesetzt wird.

8. Verfahren nach Anspruch 7, dadurch gekennzeichnet, daß die Umsetzung bei Temperaturen oberhalb 120 °C durchgeführt wird.

9. Verfahren nach einem der Ansprüche 4 bis 8, dadurch gekennzeichnet, daß die Acylierung des Zwischenproduktes mit Säurechloriden, Säureanhydriden oder Ketenen erfolgt.

10. Verfahren nach einem der Ansprüche 4 bis 9, dadurch gekennzeichnet, daß als Acylierungsmittel ein Dicarbonsäureanhydrid verwendet wird.

11. Verwendung der Verbindungen gemäß Ansprüchen 1 bis 3 und der Verfahrensprodukte gemäß Ansprüchen 4 bis 10 als Lösungsmittel, Lösungsvermittler, Gleitmittel, Weichmacher, Antistatika, als Polymerbaustein sowie als Rohstoff für Tenside und Netzmittel.

**Patentansprüche für folgende Vertragsstaaten : ES, GR**

1. Verfahren zur Herstellung von Verbindungen mit mindestens drei funktionellen Estergruppen der allgemeinen Formel:

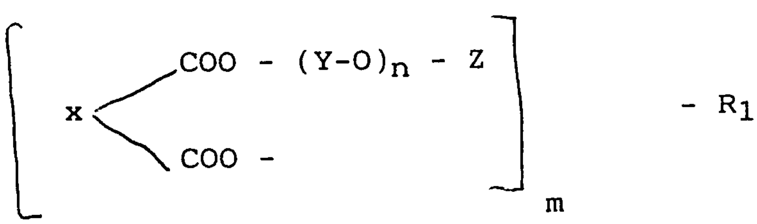

in der die verwendeten Abkürzungen die folgende Bedeutung haben:

x = $-CH_2-CH_2-$ oder $-CH=CH-$,

Y = bivalenter, gesättigter aliphatischer Kohlenwasserstoffrest, der ggfs. mit einer oder mehreren veretherten oder veresterten Hydroxygruppen substituiert ist,

Z = Acylrest -CO-R einer ein- oder zweibasigen, gesättigten oder ungesättigten aliphatischen, cycloaliphatischen oder aromatischen Carbonsäure mit 2 bis 22 C-Atomen, die gegebenenfalls einen gesättigten oder ungesättigten $C_4$-$C_{16}$-Kohlenwasserstoffrest als Substituent trägt,

$R_1$ = Rest eines ein- oder mehrwertigen gesättigten oder ungesättigten aliphatischen, cycloaliphatischen oder aromatischen Alkohols oder eine die Alkoholfunktion in der Estergruppierung übernehmende Gruppe aus einem Alkylenoxid-Addukt,

n = 1 bis 3

m = 1 bis 3, ausgenommen Ethylenglykol bis(alkylfumarate) der Formel

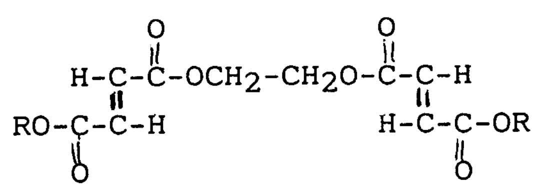

in der R eine Alkylgruppe bedeutet, dadurch gekennzeichnet, daß a) ein Dicarbonsäuremonoester mit einem Epoxid umgesetzt und die Zwischenstufe des Hydroxyalkyl-alkyl-dicarbonsäurediesters acyliert wird, oder b) ein Dicarbonsäuremonoester mit einem Alkylencarbonat in Gegenwart eines Acylierungsmittels umsetzt wird.

**2.** Verfahren nach Anspruch 1, wobei n einen Wert von 1 bis 3 aufweist.

**3.** Verfahren nach Anspruch 1 oder 2, wobei Y für eine durch Anlagerung von Alkylen- oder Cycloalkylenoxid gebildete gesättigte Gruppierung steht, die ggfs. einen oder mehrere aliphatische, aromatische oder cycloaliphatische Substituenten oder eine oder mehrere veretherte oder veresterte Hydroxygruppen trägt.

**4.** Verfahren nach Anspruch 1, dadurch gekennzeichnet, daß ein Dicarbonsäuremonoester mit einem Epoxid umgesetzt und die Zwischenstufe des Hydroxyalkyl-Alkyl-Dicarbonsäurediesters acyliert wird.

**5.** Verfahren nach Anspruch 4, dadurch gekennzeichnet, daß die Umsetzung des Dicarbonsäuremonoesters mit dem Epoxid bei Temperaturen zwischen 50 und 120 °C durchgeführt wird.

**6.** Verfahren nach Anspruch 5, dadurch gekennzeichnet, daß die Umsetzung des Dicarbonsäuremonoesters mit dem Epoxid bei Temperaturen zwischen 60 und 90 °C durchgeführt wird.

**7.** Verfahren nach Anspruch 1, dadurch gekennzeichnet, daß ein Dicarbonsäuremonoester mit einem Alkylencarbonat in Gegenwart eines Acylierungsmittels umgesetzt wird.

**8.** Verfahren nach Anspruch 7, dadurch gekennzeichnet, daß die Umsetzung bei Temperaturen oberhalb 120 °C durchgeführt wird.

**9.** Verfahren nach einem der Ansprüche 4 bis 8, dadurch gekennzeichnet, daß die Acylierung des Zwischenproduktes mit Säurechloriden, Säureanhydriden oder Ketenen erfolgt.

**10.** Verfahren nach einem der Ansprüche 4 bis 9, dadurch gekennzeichnet, daß als Acylierungsmittel ein Dicarbonsäureanhydrid verwendet wird.

**11.** Verwendung der Verfahrensprodukte gemäß Ansprüchen 1 bis 10 als Lösungsmittel, Lösungsvermittler, Gleitmittel, Weichmacher, Antistatika, als Polymerbaustein sowie als Rohstoff für Tenside und Netzmittel.

**Claims**
**Claims for the following Contracting States : AT, BE, CH, DE, FR, GB, IT, LI, LU, NL, SE**

**1.** Compounds with at least three functional ester groups of the general formula:

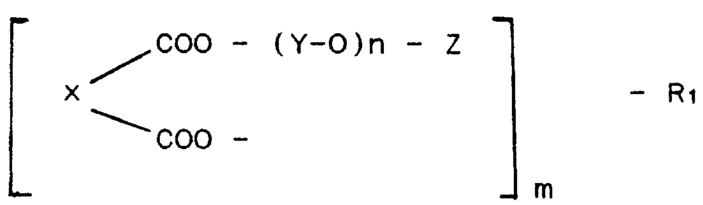

in which the abbreviations have the following meaning:

- x = $-CH_2-CH_2-$ or $-CH=CH-$,
- Y = bivalent, saturated aliphatic hydrocarbon residue optionally substituted with one or more etherified or esterified hydroxy groups,
- Z = acyl residue -CO-R of a monobasic or dibasic, saturated or unsaturated aliphatic, cycloaliphatic or aromatic carboxylic acid with 2 to 22 carbon atoms, said carboxylic acid optionally having a saturated or unsaturated $C_4$-$C_{16}$-hydrocarbon residue as substituent,
- $R_1$ = the residue of a monovalent or multivalent saturated or unsaturated aliphatic, cycloaliphatic or aromatic alcohol, or a group from an alkyleneoxide-adduct taking the alcohol function in the ester grouping,
- n = 1 to 3
- m = 1 to 3, excluding ethylene glycol bis(alkyl fumarates) of the formula:

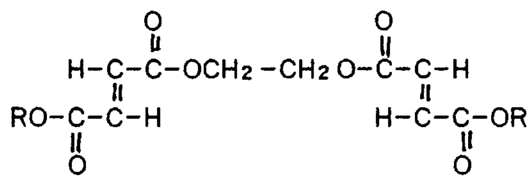

in which R means an alkyl group.

2. The compounds according to claim 1 wherein n has a value of 1 to 3.

3. The compounds according to claim 1 or 2 wherein Y represents a saturated grouping formed by the addition of alkylene- or cycloalkylene oxide, said grouping optionally having one or more aliphatic, aromatic or cycloaliphatic substituents or one or more etherified or esterified hydroxy groups.

4. A process for the production of compounds according to claim 1, characterized in that a dicarboxylic acid monoester is reacted with an epoxide and the intermediate stage of the hydroxyalkyl-alkyl-dicarboxylic acid diester is acylated.

5. The process according to claim 4 characterized in that the reaction of the dicarboxylic acid monoester with the epoxide is carried out at temperatures between 50 and 120 °C.

6. The process according to claim 5 characterized in that the reaction of the dicarboxylic acid monoester with the epoxide is carried out at temperatures between 60 and 90 °C.

7. A process for the production of compounds as defined in claim 1, characterized in that a dicarboxylic acid monoester is reacted with an alkylene carbonate in the presence of an acylating agent.

8. The process according to claim 7 characterized in that the reaction is carried out at temperatures above 120 °C.

9. The process according to any one of claims 4 to 8 characterized in that the acylation of the intermediate product is carried out with acid chlorides, acid anhydrides or ketenes.

10. The process according to any one of claims 4 to 9 characterized in that a dicarboxylic acid anhydride is used as acylating agent.

11. The use of the compounds as defined in claims 1 to 3 and of the process products according to claims 4 to 10 as solvents, solubilizers, lubricants, softeners, antistatics, structural polymer element, and as raw material for surfactants and wetting agents.

**Claims for the following Contracting States : ES, GR**

1. A process for the production of compounds with at least three functional ester groups of the general formula:

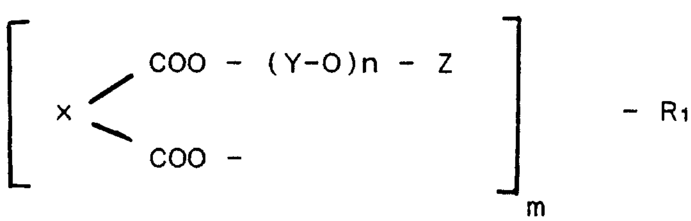

in which the abbreviations have the following meaning:

x = $-CH_2-CH_2-$ or $-CH=CH-$,

Y = bivalent, saturated aliphatic hydrocarbon residue optionally substituted with one or more etherified or esterified hydroxy groups,

Z = acyl residue -CO-R of a monobasic or dibasic, saturated or unsaturated aliphatic, cycloaliphatic or aromatic carboxylic acid with 2 to 22 carbon atoms, said carboxylic acid optionally having a saturated or unsaturated $C_4$-$C_{16}$-hydrocarbon residue as substituent,

$R_1$ = the residue of a monovalent or multivalent saturated or unsaturated aliphatic, cycloaliphatic or aromatic alcohol, or a group from an alkyleneoxide-adduct taking the alcohol function in the ester grouping,

n = 1 to 3

m = 1 to 3, excluding ethylene glycol bis(alkyl fumarates) of the formula:

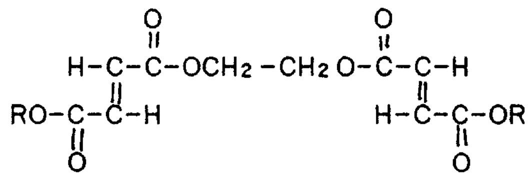

in which R means an alkyl group, characterized in that a) a dicarboxylic acid monoester is reacted with an epoxide and the intermediate stage of the hydroxyalkyl-alkyl-dicarboxylic acid diester is acylated or b) a dicarboxylic acid monoester is reacted with an alkylene carbonate in the presence of an acylating agent.

**2.** The process according to claim 1 wherein n has a value of 1 to 3.

**3.** The process according to claim 1 or 2 wherein Y represents a saturated grouping formed by the addition of alkylene- or cycloalkylene oxide, said grouping optionally having one or more aliphatic, aromatic or cycloaliphatic substituents or one or more etherified or esterified hydroxy groups.

**4.** The process according to claim 1 characterized in that a dicarboxylic acid monoester is reacted with an epoxide and the intermediate stage of the hydroxyalkyl-alkyl-dicarboxylic acid diester is acylated.

**5.** The process according to claim 4 characterized in that the reaction of the dicarboxylic acid monoester with the epoxide is carried out at temperatures between 50 and 120 °C.

**6.** The process according to claim 5 characterized in that the reaction of the dicarboxylic acid monoester with the epoxide is carried out at temperatures between 60 and 90 °C.

**7.** The process according to claim 1 characterized in that a dicarboxylic acid monoester is reacted with an alkylene carbonate in the presence of an acylating agent.

**8.** The process according to claim 7 characterized in that the reaction is carried out at temperatures above 120 °C.

**9.** The process according to any one of claims 4 to 8 characterized in that the acylation of the intermediate product is carried out with acid chlorides, acid anhydrides or ketenes.

**10.** The process according to any one of claims 4 to 9 characterized in that a dicarboxylic acid anhydride is used as acylating agent.

**11.** The use of the process products according to claims 1 to 10 as solvents, solubilizers, lubricants, softeners, antistatics, structural polymer element, and as raw material for surfactants and wetting agents.

**Revendications**

**Revendications pour les Etats contractants suivants : AT, BE, CH, DE, FR, GB, IT, LI, LU, NL, SE**

**1.** Composés comprenant au moins trois radicaux ester fonctionnels de la formule générale :

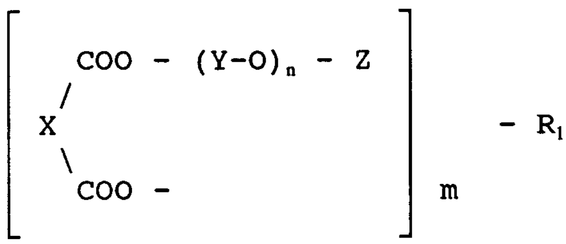

dans laquelle les abréviations utilisées ont les significations suivantes :

X = $-CH_2-CH_2-$ ou $-CH=CH-$,

Y = radical hydrocarboné aliphatique, saturé, bivalent, qui est éventuellement substitué par un ou plusieurs radicaux hydroxyle éthérifiés ou estérifiés,

Z = radical acyle -CO-R d'un acide carboxylique aromatique, cycloaliphatique, aliphatique, saturé ou insaturé, monobasique ou dibasique, comportant de 2 à 22 atomes de carbone, qui porte éventuellement un radical hydrocarboné en $C_4$ à $C_{16}$, saturé ou insaturé, à titre de substituant,

$R_1$ = le reste d'un alcool aromatique, cycloaliphatique ou aliphatique, saturé ou insaturé, monohydroxylé ou polyhydroxylé, ou un groupe reprenant la fonction alcool dans le groupement ester, constitué d'un adduit d'oxyde d'alkylène,

n = 1 à 3,

m = 1 à 3, à l'exclusion d'éthylèneglycol bis(fumarates d'alkyle) de la formule :

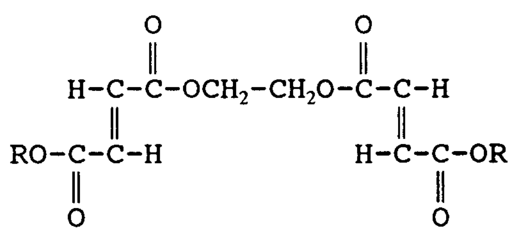

dans laquelle R représente un radical alkyle.

**2.** Composés suivant la revendication 1, caractérisés en ce que n a une valeur qui varie de 1 à 3.

**3.** Composés suivant la revendication 1 ou 2, caractérisés en ce que Y représente un groupement saturé formé par addition d'un oxyde d'alkylène ou de cycloalkylène, qui porte éventuellement un ou plusieurs substituants aliphatiques, aromatiques ou cycloaliphatiques, ou un ou plusieurs radicaux hydroxyle éthérifiés ou estérifiés.

**4.** Procédé de préparation de composés suivant la revendication 1, caractérisé en ce que l'on fait réagir un monoester d'un acide dicarboxylique avec un époxyde et on acyle le produit intermédiaire du dicarboxylate d'hydroxyalkyle et d'alkyle.

**5.** Procédé suivant la revendication 4, caractérisé en ce que l'on entreprend la réaction du monoester de l'acide dicarboxylique avec l'époxyde à des températures de 50 à 120 °C.

**6.** Procédé suivant le revendication 5, caractérisé en ce que l'on entreprend la réaction du monoester de l'acide dicarboxylique avec l'époxyde à une température qui varie de 60 à 90 °C.

**7.** Procédé de préparation de composés suivant la revendication 1, caractérisé en ce que l'on fait réagir un monoester d'acide dicarboxylique avec un carbonate d'alkylène en présence d'un agent d'acylation.

**8.** Procédé suivant la revendication 7, caractérisé en ce que l'on entreprend la réaction à des températures supérieures à 120 °C.

**9.** Procédé suivant l'une quelconque des revendications 4 à 8, caractérisé en ce que l'acylation du produit intermédiaire s'effectue avec des chlorures d'acides, des anhydrides d'acides ou des cétènes.

**10.** Procédé suivant l'une quelconque des revendications 4 à 9, caractérisé en ce que l'on utilise un anhydride d'acide dicarboxylique à titre d'agent d'acylation.

**11.** Utilisation des composés suivant l'une quelconque des revendications 1 à 3 et des produits obtenus par le procédé suivant l'une quelconque des revendications 4 à 10 à titre de solvants, d'agents conférant de la solubilité, de lubrifiants, de plastifiants, d'antistatiques, d'éléments constitutifs de polymères, comme aussi de matières premières pour des produits tensioactifs ou surfactifs et des agents mouillants.

**Revendications pour les Etats contractants suivants : ES, GR**

**1.** Procédé de préparation de composés comprenant au moins trois radicaux ester fonctionnels de la formule générale :

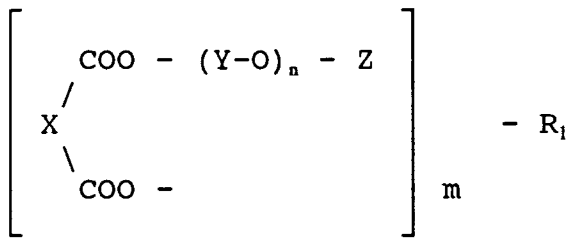

dans laquelle les abréviations utilisées ont les significations suivantes :

X = $-CH_2-CH_2-$ ou $-CH=CH-$,

Y = radical hydrocarboné aliphatique, saturé, bivalent, qui est éventuellement substitué par un ou plusieurs radicaux hydroxyle éthérifiés ou estérifiés,

Z = radical acyle -CO-R d'un acide carboxylique aromatique, cycloaliphatique, aliphatique, saturé ou insaturé, monobasique ou dibasique, comportant de 2 à 22 atomes de carbone, qui porte éventuellement un radical hydrocarboné en $C_4$ à $C_{16}$, saturé ou insaturé, à titre de substituant,

$R_1$ = le reste d'un alcool aromatique, cycloaliphatique ou aliphatique, saturé ou insaturé, monohydroxylé ou polyhydroxylé, ou un groupe reprenant la fonction alcool dans le groupement ester, constitué d'un adduit d'oxyde d'alkylène,

n = 1 à 3,

m = 1 à 3, à l'exclusion d'éthylèneglycol bis(fumarates d'alkyle) de la formule :

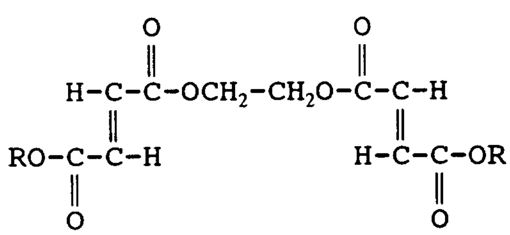

dans laquelle R représente un radical alkyle,

caractérisé en ce que (a) on fait réagir un monoester d'acide dicarboxylique avec un époxyde et on

acyle le produit intermédiaire du dicarboxylate d'hydroxyalkyle et d'alkyle, ou (b) on fait réagir un monoester d'acide dicarboxylique avec un carbonate d'alkylène en présence d'un agent d'acylation.

**2.** Procédé suivant la revendication 1, caractérisé en ce que n a une valeur qui varie de 1 à 3.

**3.** Procédé suivant la revendication 1 ou 2, caractérisé en ce que Y représente un groupement saturé formé par addition d'un oxyde d'alkylène ou de cycloalkylène, qui porte éventuellement un ou plusieurs substituants aliphatiques, aromatiques ou cycloaliphatiques, ou un ou plusieurs radicaux hydroxyle éthérifiés ou estérifiés.

**4.** Procédé suivant la revendication 1, caractérisé en ce que l'on fait réagir un monoester d'un acide dicarboxylique avec un époxyde et on acyle le produit intermédiaire du dicarboxylate d'hydroxyalkyle et d'alkyle.

**5.** Procédé suivant la revendication 4, caractérisé en ce que l'on entreprend la réaction du monoester de l'acide dicarboxylique avec l'époxyde à des températures de 50 à 120 °C.

**6.** Procédé suivant le revendication 5, caractérisé en ce que l'on entreprend la réaction du monoester de l'acide dicarboxylique avec l'époxyde à une température qui varie de 60 à 90 °C.

**7.** Procédé suivant la revendication 1, caractérisé en ce que l'on fait réagir un monoester d'acide dicarboxylique avec un carbonate d'alkylène en présence d'un agent d'acylation.

**8.** Procédé suivant la revendication 7, caractérisé en ce que l'on entreprend la réaction à des températures supérieures à 120 °C.

**9.** Procédé suivant l'une quelconque des revendications 4 à 8, caractérisé en ce que l'acylation du produit intermédiaire s'effectue avec des chlorures d'acides, des anhydrides d'acides ou des cétènes.

**10.** Procédé suivant l'une quelconque des revendications 4 à 9, caractérisé en ce que l'on utilise un anhydride d'acide dicarboxylique à titre d'agent d'acylation.

**11.** Utilisation des produits obtenus par mise en oeuvre du procédé suivant l'une quelconque des revendications 1 à 10 à titre de solvants, d'agents conférant de la solubilité, de lubrifiants, de plastifiants, d'antistatiques, d'éléments constitutifs de polymères, comme aussi de matières premières pour des produits tensioactifs ou surfactifs et des agents mouillants.